# EUROPEAN PATENT APPLICATION

(11) **EP 2 889 294 A1**
(43) Date of publication of application: **01.07.2015**
(21) Application number: 13831580.9
(22) Date of filing: 16.01.2013
(51) Int. Cl.: C07D 339/04, C07D 409/04, A61K 31/385, A61K 31/4365, A61P 9/10, A61P 7/02

(54) **3H-1,2-DITHIOCYCLOPENTENE-3-THIOKETONE COMPOUNDS AND APPLICATION THEREOF**

(30) Priority: 21.08.2012 CN 201210298524
(71) Applicant: Soochow University, Jiangsu 215123 (CN)
(72) Inventor: CHENG, Jian, Suzhou Jiangsu 215123 (CN); AO, Guizhen, Suzhou Jiangsu 215123 (CN); JIA, Jia, Suzhou Jiangsu 215123 (CN); YANG, Jin, Nanjing, Jiangsu 210009 (CN)
(74) Representative: Tappe, Udo
(86) International application number: PCT/CN2013/070508
(87) International publication number: WO 2014/029197

(57) **Abstract**

The present invention relates to the field of medicaments, and in particular relates to 3H-1,2-dithiocyclopentene-3-thione compounds and application thereof. 3H-1,2-dithiocyclopentene-3-thione compounds disclosed by the invention have new structures shown in formula I or formula II. Proved by experiments, 3H-1,2-dithiocyclopentene-3-thione compounds can directly protect neurons in a cellular model, and can significantly restrain excessive inflammatory responses of brain inflammatory cells; and by using 3H-1,2-dithiocyclopentene-3-thione, focal ischemic cerebral infarct volumes of mice can be remarkably reduced in an animal model. Therefore, the invention provides the application of 3H-1,2-dithiocyclopentene-3-thione compounds and pharmaceutically acceptable salts thereof in the preparation of medicaments for preventing or treating cerebral apoplexy diseases.

## Description

The present application claims the priority of Chinese patent application No. 201210298524.4, filed on 21, August, 2012, to the Patent Office of the People's Republic of China, and titled "3H-1,2-dithiocyclopentene-3-thione compounds and application thereof", which is hereby incorporated by reference in its entirety.

### FIELD OF THE INVENTION

The present invention relates to the pharmaceutical filed, and in particular to 3H-1,2-dithiocyclopentene-3-thione compounds and application thereof, and particularly to the application of 3H-1,2-dithiocyclopentene-3-thione compounds in preparation of medicines for the treatment or prevention of cerebral apoplexy diseases.

### BACKGROUND OF THE INVENTION

Cerebral apoplexy is the third leading cause of death in the population all around the world, and is the primary reason that results in disability in adults. According to the statistical results, there are 500 to 750 thousand of people attacked by stroke per year in the United States; while in China, the number of people suffered from cerebral apoplexy is as much as 2.5 million per year, and the number of people died is 1.6 million per year. At present, cerebral apoplexy has already surpassed coronary occlusion to become the leading cause of death in china (Liu L, et al., Stroke. 2011; 42:3651-3654). In one aspect, cerebral apoplexy has high death rate, while in another aspect, as high as 75% patients who suffer from apoplexy may have sequelae, such as damage to neurobehavioral functions and cognitive impairment, resulting in heavy economical burden to the society and families.

Cerebral apoplexy is primarily divided into two types. More than 70% cases of cerebral apoplexy belong to ischemic cerebral apoplexy, which refers to ischemic brain injury thus resulted from cerebrovascular blockages induced by various types of thrombosis in different brain regions, wherein the most common one is middle cerebral artery occlusion. In addition, no more than 30% cases of cerebral apoplexy are caused by cerebral hemorrhage induced by rupture of cerebral vessels, which finally results in insufficient blood supply in brain and ischemic brain injury. Cerebral ischemia is also one of the outcomes of sudden cardiac arrest.

With respect to the severity and the extent of injury of cerebral apoplexy, the existing therapies for cerebral apoplexy are still limited. For ischemic cerebral apoplexy, for example, the only globally recognized therapeutic measure is thrombolysis at the acute stage using tissue-type plasminogen activator. However, the patients suitable for being treated withtissue-type plasminogen activator account for no more than 5% of the total patients suffered from cerebral ischemia, due to short therapeutic time window and severe side effects (such as cerebral hemorrhage and/or aggravated excitatory neurotoxicity) of tissue-type plasminogen activator.

A series of cellular and molecular mechanisms have been shown to determine the death or survival of neural cells following cerebral apoplexy. At present, it has been found that the primary mechanisms responsible for the death or injury of neurons by cerebral ischemia include: neuron injury due to excessive release of excitatory amino acid (such as glutamate), the injury mechanism caused by excessive production of free radical, and the mechanism of neuronal injury resulted from over-activated inflammatory responses in brain following cerebral apoplexy. These pathological injury mechanisms are of great importance to the research and development of the therapeutic medicines for cerebral apoplexy. For example, edaravone is a type of free radical scavenger, which has been approved for the treatment of ischemic cerebral apoplexy in China and Japan. However, since the exact curative effect of edaravone on cerebral apoplexy is still under investigation (Lapchak PA, et al., Expert Opin Pharmacother., 2010;11:1753-1763), edaravone has still not been approved as the clinical therapeutic medicine for cerebral apoplexy in Europe and America at present. In brief, there are still few therapeutic medicines at present for cerebral apoplexy, with respect to its high incidence and severity. As a result, it is important to develop novel medicines for the treatment and prevention of cerebral apoplexy, based on it's the pathological mechanisms.

### SUMMARY OF THE INVENTION

Based on the content mentioned above, the objective of the present invention is to provide 3H-1,2-dithiocyclopentene-3-thione compounds and their applications in preparation of medicines for the treatment or prevention of cerebral apoplexy diseases.

The 3H-1,2-dithiocyclopentene-3-thione compounds of the present invention have the structure of formula I or formula II,

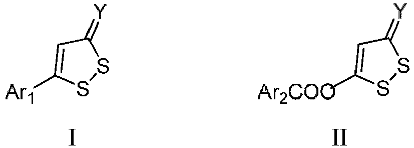

Wherein Y is selected from S, O or NHOH; Ar₁ is selected from naphthyl, phenyl, thiophene, furan, pyrrole, imidazole, thiazole, pyrazole, and pyridine; the substituent on Ar₁ is a mono-substituent, di-substituent or tri-substituent, and the substituents are independently from each other selected from -H, -OH, -NH₂, -X, -SO₂CH₃, -SO₂NH₂, -CN, -NO₂, -COOH, Ar- or R-, RO-, XR-, RNH-, NH₂R-, NO₂O-R-, or HOOC-R-, wherein X is F, Cl, Br, or I, and R represents an alkyl of 1-8 carbon atoms; Ar₂COO is selected from the carboxylic residue from nicotinic acid, aspirin, diclofenac, ketoprofen or ibuprofen.

Among others, thiophene, furan, pyrrole, imidazole, thiazole, pyrazole and pyridine are bioisosteres. Based on the principles of pharmaceutical chemistry, thiophene, furan, pyrrole, imidazole, thiazole, pyrazole and pyridine have similar pharmacological activities.

Preferably, the 3H-1,2-dithiocyclopentene-3-thione compounds are selected from 5-*p*-hydroxyphenyl-3H-1,2-dithiacyclopentene-3-thione, 5-*p*-fluorophenyl-3H-1,2-dithiacyclopentene-3-thione, 5-(2-thienyl)-3H-1,2-dithiacyclopentene-3-thione, 5-*p*- methylphenyl -3H-1,2-dithiacyclopentene-3-thione, 5-(2,4-dichloro-5-fluorophenyl)-3H-1,2-dithiacyclopentene-3-thione, 5-(3,4-methylenedioxyphenyl)-3H-1,2-dithiacyclopentene-3-thione, 5-(2,4-dimethoxyphenyl)-3H-1,2-dithiacyclopentene-3-thione, 5-*p*-methoxyphenyl-3H-1,2-dithiacyclopentene-3-thione, 5-phenyl-3H-1,2-dithiacyclopentene-3-thione, 5-(2-naphthyl)-3H-1,2-dithiacyclopentene-3-thione, 4-(3H-1,2-dithio-3-thioketone 5-yl)phenyl 4-isobutyl-α-methyl-phenylacetate, 4-(3H-1,2-dithio-3-thioketone-5-yl)phenyl 2-acetoxy benzoate, 4-(3H-1,2-dithio-3-thioketone-5-yl)phenyl pyridine-3-carboxylate, 4-(3H-1,2-dithio-3-thioketone-5-yl)phenyl 3-benzoyl-phenylacetate or 4-(3H-1,2-dithio-3-thioketone-5-yl)-phenyl 2-[(2,6-dichlorophenyl)amino]phenylacetate.

Glutamate is an important excitatory neurotransmitter in central nervous system. s Extracellular glutamate remarkably enhanced following cerebral ischemia, cerebral hemorrhage, and brain trauma. One of the primary mechanisms responsible for the neuronal damage resulted from the excessive glutamate is oxidative stress injury. In the present invention, neuronal oxidative stress injury is induced in HT22 hippocampal neuron cell line by treatment of HT22 cells with glutamate, and MTT method is used to investigate the protective effect of 3H-1,2-dithiocyclopentene-3-thione compounds on neurons against glutamate induced-oxidative stress injury. As shown in Table 1, following glutamate treatment, the survival rate of HT22 cells co-treated with the 3H-1,2-dithiocyclopentene-3-thione compounds prepared in the Examples of the present invention at the final concentrations from 1 to 100µM is greater than 42% , which is much higher than the survival rate of control HT22 cells co-treated with vehicle (24%). These results indicates that 3H-1,2-dithiocyclopentene-3-thione compounds exert the direct protection on neurons against the glutamate-induced oxidative stress.

It has been shown that a great amount of damage-associated molecular pattern (DAMP) molecules are released in the brain after cerebral apoplexy, and the over-activation of the brain-resident inflammatory cells (microglia) by DAMP is one of the important inflammatory mechanisms responsible for brain injury following cerebral apoplexy. In the present invention, microglia is over-activated by lipopolysaccharide (LPS), the well-recognized DAMP for the activation of inflammatory cells.. In this model, the inhibitory effect of 3H-1,2-dithiocyclopentene-3-thione compounds on the over-activation of brain-resident inflammatory cells is indicated by the decrease in LPS-induced nitrite from microglia. The results showed that the inflammatory response of microglia induced by lipopolysaccharide was significantly inhibited by the 3H-1,2-dithiocyclopentene-3-thione compounds YC1-1, YC1-8 and YC2-2 prepared in the examples of the present invention at final concentrations from 10 to 100µM.

In the present invention, the therapeutic effect of 3H-1, 2-dithiocyclopentene-3-thione compounds against ischemic brain injury was investigated in the mouse middle cerebral artery occlusion model by measuring the cerebral infarction volumes using TTC staining method. The results indicated that after 24 h middle cerebral artery occlusion, the infarction volumes in cerebral cortex, striatum and hemisphere can be significantly reduced by injection of 100 mg/kg and 50 mg/kg 3H-1,2-dithiocyclopentene-3-thione compound YC1-8, as compared to the mice in the control group treated with vehicle, which suggested that there was a remarkable protective effect of YC1-8 against the ischemic brain injury resulted from middle cerebral artery occlusion.

In conclusion, there is a direct protective effect of the 3H-1,2-dithiocyclopentene-3-thione compounds described herein on neurons against the oxidative stress injury due to ischemia, and the inflammatory responses of intracerebral inflammatory cells can be significantly inhibited, and the focal cerebral infarction volumes can be remarkably reduced in the animal model by the compounds. As a result, cerebral apoplexy can be prevented and treated by the 3H-1,2-dithiocyclopentene-3-thione compounds described herein based on the effects of these compound on the two primary cerebral apoplexy-related pathological injury mechanisms, including "neural oxidative stress injury resulted from excessive release of glutamate after cerebral ischemia" and "overactivation of intracerebral inflammatory cells". Thus, the present invention provides application of 3H-1,2-dithiocyclopentene-3-thione compounds and the pharmaceutically acceptable salts thereof in preparation of medicines for the prevention or treatment of cerebral apoplexy diseases.

In some embodiments, the 3H-1,2-dithiocyclopentene-3-thione compounds have the structure of formula I,

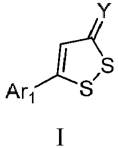

Wherein Y is selected from S, O or NHOH; Ar₁ is selected from naphthyl, phenyl, thiofuran, furan, pyrrole, imidazole, thiazole, pyrazole, and pyridine; the substituent on Ar₁ is a mono-substituent, di-substituent or tri-substituent, and the substituents are independently from each other selected from -H, -OH, -NH₂, -X, -SO₂CH₃, -SO₂NH₂, -CN, -NO₂, -COOH, Ar- or R-, RO-, XR-, RNH-, NH₂R-, NO₂OR-, HOOCR-, wherein X is F, Cl, Br, or I, and R represents an alkyl of 1-8 carbon atoms.

In some embodiments, the 3H-1,2-dithiocyclopentene-3-thione compounds have the structure of formula II,

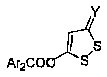

Ar₂COO is selected from the carboxylic residue from niacin, aspirin, diclofenac, ketoprofen or ibuprofen.

Further, the 3H-1,2-dithiocyclopentene-3-thione compounds described herein are selected from, 5-*p*-hydroxyphenyl-3H-1,2-dithiacyclopentene-3-thione, 5-*p*-fluorophenyl-3H-1,2-dithiacyclopentene-3-thione, 5-(2-thienyl)-3H-1,2-dithiacyclopentene-3-thione, 5-*p*- methylphenyl -3H-1,2-dithiacyclopentene-3-thione, 5-(2,4-dichloro-5-fluorophenyl)-3H-1,2-dithiacyclopentene-3-thione, 5-(3,4-methylenedioxyphenyl)-3H-1,2-dithiacyclopentene-3-thione, 5-(2,4-dimethoxyphenyl)-3H-1,2-dithiacyclopentene-3-thione, 5-*p*-methoxyphenyl-3H-1,2-dithiacyclopentene-3-thione, 5-phenyl-3H-1,2-dithiacyclopentene-3-thione, 5-(2-naphthyl)-3H-1,2-dithiacyclopentene-3-thione, 4-(3H-1,2-dithiacyclopentene-3-thioketone 5-yl)phenyl 4-isobutyl-α-methyl-phenylacetate, 4-(3H-1,2-dithio-3-thioketone-5-yl)phenyl 2-acetoxy benzoate, 4-(3H-1,2-dithio-3-thioketone-5-yl)phenyl pyridine-3-carboxylate , 4-(3H-1,2-dithio-3-thioketone-5-yl)phenyl 3-benzoyl-phenylacetate or 4-(3H-1,2-dithio-3-thioketone-5-yl)-phenyl 2-[(2,6-dichlorophenyl)amino]phenylacetate.

The cerebral apoplexy described herein primarily refers to the cerebral apoplexy resulted from cerebral embolism or cerebral hemorrhage. Furthermore, the cerebral apoplexy is selected from one or more of cerebral thrombosis, cerebral embolism, cerebral infarction, lacunar infarction, transient ischemic attack, cerebral hemorrhage, cerebral arteriosclerosis, diabetes-induced cerebrovascular complications or cerebral ischemia due to sudden cardiac arrest.

The present invention also provides a pharmaceutical formulation for the prevention or treatment of cerebral apoplexy diseases, which comprises effective doses of 3H-1,2-dithiocyclopentene-3-thione compounds or the pharmaceutically acceptable salts thereof and the pharmaceutically acceptable adjuvants. The 3H-1,2-dithiocyclopentene-3-thione compounds or the pharmaceutically acceptable salts thereof can be directly or indirectly added to various types of pharmaceutically acceptable adjuvants, such as disintegrant, lubricant, emulsifier, or adhesive etc., that is demanded for the preparation of different formulations via routine pharmaceutical preparation methods by the one of skills in the art to prepare commonly used formulations, including, but not limited to capsule, microcapsule, tablet, granule, dispersible powder, injection, liposome, oral solution, intravenous injection, intramuscular injection or the formulation that can be directly applied to the site of cerebral ischemia.

In the pharmaceutical formulations described herein, 0.05 wt% to 90 wt% of 3H-1,2-dithiocyclopentene-3-thione compounds or the pharmaceutically acceptable salts thereof can be contained, and more commonly, in some embodiments, about 15 wt% to 60 wt% of 3H-1,2-dithiocyclopentene-3-thione compounds or the pharmaceutically acceptable salts thereof can be contained.

The pharmaceutical formulations described herein can be administrated by the following routes, including oral, rectal, nasal, local, buccal, sublingual, and parenteral administration, such as subcutaneous, intravenous, intramuscular, intraperitoneal, intrathecal, intraventricular, intrasternal, or intracranial injection or infusion, or administration by a explanted container, wherein oral administration, intramuscular injection, intraperitoneal and intravenous administration are preferable.

The dosage and application method of the formulations described herein for the prevention or treatment of cerebral apoplexy are based on many factors, including the age, body weight, gender, natural health condition, and nutritional quality of the patients, the activity, medication duration, and metabolism velocity of the compounds, the severity of the diseases, and the subjective judgment of the physician. Based on the factors mentioned above, the dosage and application method can be easily determined by the one of ordinary skills in the art. Generally, the pharmaceutical formulations described herein can be administrated at a dosage from 0.005 to 5000 mg/kg/day, or at a dosage beyond the above range based on the severity of the diseases or different formulations.

### BRIEF DESCRIPTION OF THE FIGURES

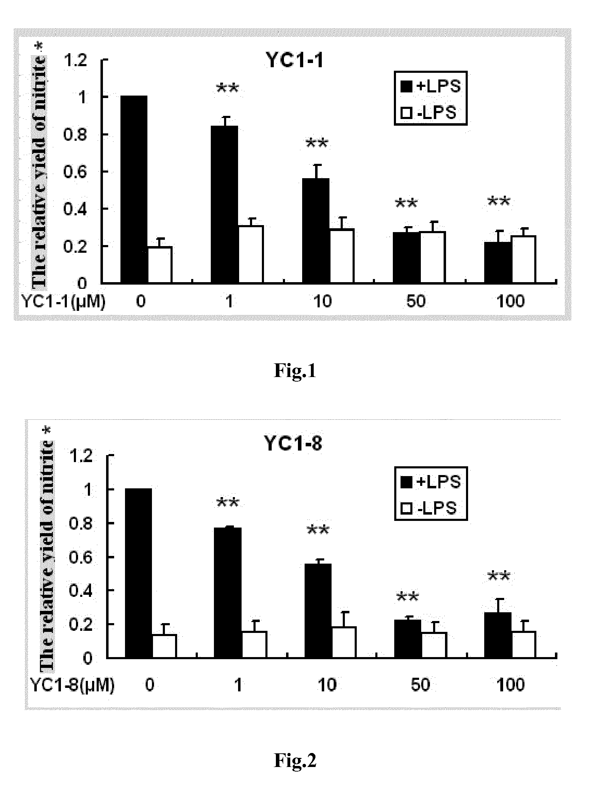

Fig. 1 shows the statistical graph of the inhibitory effect of compound YC1-1 synthesized in Example 1 on the lipopolysaccharide (LPS)-induced inflammatory response of the intracerebral inflammatory cells (microglia cell line BV2); wherein the relative yield of nitrite produced in the cells of the control group treated with LPS alone is 1; ** indicates the comparison between the relative yield of nitrite in the cells treated with different concentrations of YC1-1 plus LPS and that in the cells treated with LPS alone; p<0.01 means very significant difference;
Fig. 2 shows the statistical graph of the inhibitory effect of compound YC1-8 synthesized in Example 8 on the lipopolysaccharide (LPS) induced inflammatory response of the intracerebral inflammatory cells (microglia cell line BV2); wherein the relative yield of nitrite produced in the cells of the control group treated with LPS alone is 1; ** indicates the comparison between the relative yield of nitrite in the cells treated with different concentrations of YC1-8 plus LPS and that in the cells treated with LPS alone; p<0.01 means very significant difference;

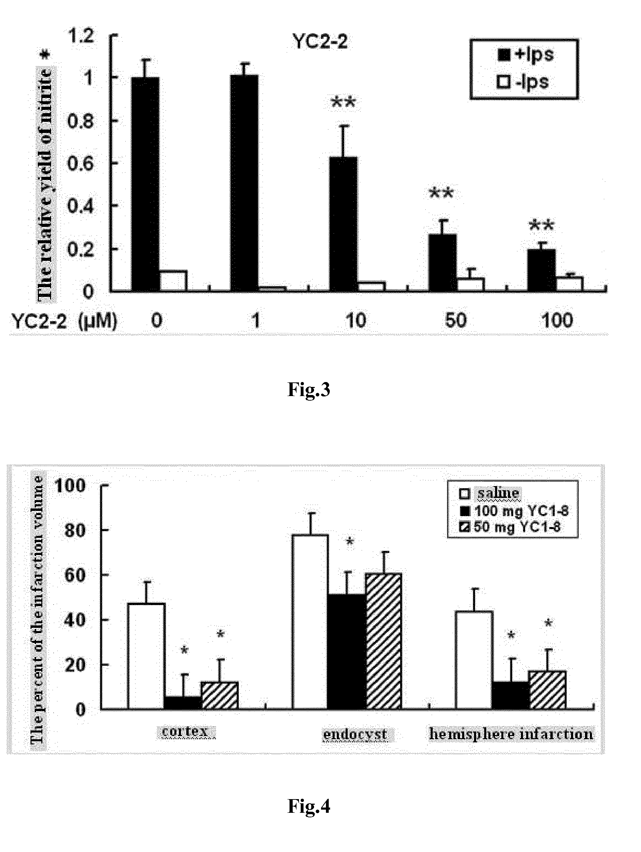

Fig. 3 shows the statistical graph of the inhibitory effect of compound YC2-2 synthesized in Example 12 on the lipopolysaccharide (LPS) induced inflammatory response of the intracerebral inflammatory cells (microglia cell line BV2); wherein the relative yield of nitrite produced in the cells of the control group treated with LPS alone is 1; ** indicates the comparison between the relative yield of nitrite in the cells treated with different concentrations of YC2-2 plus LPS and that in the cells treated by LPS alone; p<0.01 means very significant difference;
Fig. 4 shows the protective effect of compound YC1-8 of Example 8 on the brain infarct damage in the mouse middle cerebral artery occlusion model; infarct volumes are expressed as the percent of the corresponding contralateral structures; * indicates comparison to the percent of infarct volume in corresponding regions of brain of the mice treated with vehicle; p<0.05 means significant difference.

### DETAILED EMBODIMENTS

In the embodiments of the present invention, the application of 3H-1, 2-dithiocyclopentene-3-thione compounds is disclosed. The present invention can be implemented by properly modifying the processing parameters by the one of skills in the art with reference to the content herein. Particularly, it should be noted that all similar replacements and modifications are apparent to the one of skills in the art, all of which are regarded to be included in the present invention. The application of the present invention has been described by preferred Examples, and it is apparent that modification, or proper change and the combination thereof can be made to the application described herein by those skilled in the art, without departing from the content, spirit and scope of the invention, in order to achieve and apply the techniques disclosed in the present invention.

For better understanding of the present invention, it will be further explained with reference to specific Examples below.

### Example 1: Synthesis of compound 5-p-hydroxyphenyl-3H-1,2-dithiacyclopentene-3-thione (YC1-1)

Methyl *p*-hydroxybenzoyl acetate (307 mg, 1.89 mmol), phosphorus pentasulfide (420 mg, 1.9 mmol), and sulfur (56 mg, 0.93 mmol) were added to 20 ml toluene. After heated to reflux for 5 h, the reaction was finished. Subsequently, the mixture was filtered and the filtrate was concentrated. It was then developed by a column chromatography (petroleum ether : acetone = 10:1) to abtain a red solid (134 mg, yield of 40%). m.p. 191.3-192.1°C. ¹H NMR (400MHz, DMSO-d₆), δ(ppm):□ 10.46 (s, 1H, OH), 7.78 (d, 2H, *J* = 8.7 Hz, ArH), 7.68 (s, 1H, =CH), 6.90 (d, 2H, *J* = 8.7 Hz, ArH). HR-MS: Calcd. For C₉H₆OS₃[M+H]⁺: 226.9654, Found: 226.9645.

### Example 2: Synthesis of 5-p-fluorophenyl-3H-1,2-dithiacyclopentene-3-thione (YC1-2)

Based on the synthesis method of YC1-1, YC1-2 was obtained using methyl *p*-fluorobenzoyl acetate, phosphorus pentasulfide and sulfur, with a yield of 34%. m.p. 108.3-110.0 °C. ¹HNMR(400Hz,CDCl₃), δ(ppm):7.66(s, 2H, ArH), 7.37(s, 1H, =CH), 7.18(s, 2H, ArH). ¹³C-NMR (400MHz, CDCl₃), δ(ppm): 215.398, 171.482, 166.623, 163.247, 135.893, 129.124, 129.007, 117.060, 116.765. HR-MS: Calcd. For C₉H₅FS₃[M+H]⁺: 228.9610, Found: 228.9610.

### Example 3: Synthesis of 5-(2-thienyl)-3H-1,2-dithiacyclopentene-3-thione (YC1-3)

Based on the synthesis method of YC1-1, YC1-3 was obtained using methyl 2-thenoyl acetate, phosphorus pentasulfide and sulfur, with a yield of 36%. m.p. 119.2-120.9°C. ¹HNMR(400Hz,CDCl₃), δ(ppm): 7.59(s, 1H, =CH), 7.54(s, 1H, ArH), 7.32(s, 1H, ArH), 7.15(s, 1H, ArH). ¹³C-NMR (400MHz, CDCl₃), δ(ppm): 214.456, 165.134, 134.629, 133.866, 131.083, 129.247, 129.065. HR-MS: Calcd. For C₇H₇S₄[M+H]⁺: 216.9269, Found: 216.9268.

### Example 4: Synthesis of 5- p-methylphenyl -3H-1,2-dithiacyclopentene-3-thione (YC1-4)

Based on the synthesis method of YC1-1, YC1-4 was obtained using methyl *p*-methyl benzoyl acetate, phosphorus pentasulfide and sulfur, with a yield of 43%. m.p. 114.3-115.6°C. ¹HNMR(400Hz, CDCl₃), δ(ppm): 7.52(d, 2H, J=8.1Hz, ArH), 7.39(s, 1H, =CH), 7.26(d, 2H, J=7.9Hz, ArH), 2.46(s, 3H, CH₃). ¹³C-NMR (400MHz, CDCl₃), δ(ppm): 215.481, 173.456, 143.354, 135.480, 130.498, 129.024, 126.984, 21.842. HR-MS: Calcd. For C₁₀H₈S₃[M+H]⁺: 224.9861, Found: 224.9860.

### Example 5: Synthesis of 5-(2,4-dichloro-5-fluorophenyl)-3H-1,2-dithiacyclopentene-3-thione (YC1-5)

Based on the synthesis method of YC1-1, YC1-5 was obtained using methyl 2,4-dichloro-5-fluoro-benzoylacetate, phosphorus pentasulfide and sulfur, with a yield of 49%. m.p. 157.9-159.3°C . ¹H-NMR(400MHz, CDCl₃), δ(ppm): 7.62(d, 1H, J=6.6Hz, ArH), 7.38(d, 1H, J=8.6Hz, ArH), 7.28(s, 1H, =CH,). ¹³C-NMR (400MHz, CDCl₃), δ(ppm): 215.494, 166.443, 158.348, 140.790, 132.980, 130.170, 128.425, 125.352, 118.781. HR-MS: Calcd. For C₉H₃Cl₂FS₃[M+H]⁺: 296.8831, Found: 296.8840.

### Example 6: Synthesis of 5-(3,4-methylenedioxyphenyl)-3H-1,2-dithiacyclopentene-3-thione (YC1-6)

Based on the synthesis method of YC1-1, YC1-6 was obtained using methyl 3,4-methylenedioxy benzoylacetate, phosphorus pentasulfide and sulfur, with a yield of 24%. m.p. 200.1-201.4°C. ¹H-NMR(400MHz, CDCl₃), δ(ppm): 7.36(s, 1H, =CH), 7.24(dd, 1H, J=1.8, 8.1Hz, ArH), 7.10(d, 1H, J=1.7Hz, ArH), 6.90(d, 1H, J=8.1Hz, ArH), 6.09(s, 2H, CH₂). HR-MS: Calcd. For C₁₀H₆O₂S₃[M+H]⁺:254.9603, Found: 254.9602.

### Example 7: Synthesis of 5-(2,4-dimethoxyphenyl)-3H-1,2-dithiacyclopentene-3-thione (YC1-7)

Based on the synthesis method of III1, YC1-7 was obtained using methyl 2,4-dimethoxy benzoylacetate, phosphorus pentasulfide and sulfur, with a yield of 35%. m.p. 135.5-136.7°C. ¹H-NMR(400MHz, CDCl₃), δ(ppm): 7.66(s, 1H, ArH), 7.64(d, 1H, J=8.8Hz, ArH), 6.61(d, 1H, J=8.7Hz, ArH), 6.55(s, 1H, =CH), 3.96(s, 3H, CH₃), 3.89(s, 3H, CH₃). HR-MS: Calcd. For C₁₁H₁₁ O₂S₃ [M+H]⁺: 270.9921, Found: 270.9904.

### Example 8: Synthesis of 5-p-methoxyphenyl-3H-1,2-dithiacyclopentene-3-thione (YC1-8)

Based on the synthesis method of YC1-1, YC1-8 was obtained using methyl *p*-methoxybenzoyl acetate, phosphorus pentasulfide and sulfur, with a yield of 49%. m.p. 110.2-111.5□. ¹H NMR (400MHz, DMSO-d₆), δ(ppm): 7.62 (d, 2H, *J* = 8.9 Hz, ArH), 7.40 (s, 1H, =CH), 6.98 (d, 2H, *J* = 8.8 Hz, ArH), 3.88 (s, 3H, OCH₃). HR-MS: Calcd. For C₁₀H₈OS₃[M+H]⁺: 240.9816, Found: 240.9811.

### Example 9: Synthesis of 5-phenyl-3H-1,2-dithiacyclopentene-3-thione (YC1-9)

Based on the synthesis method of YC1-1, YC1-9 was obtained using methyl benzoylacetate, phosphorus pentasulfide and sulfur, with a yield of 37%. m.p. 123.5-124.4°C. ¹H-NMR(400MHz, CDCl₃), δ(ppm): 7.67(d, 2H, J=7.3Hz, ArH), 7.57(t, 1H, J=7.42Hz, ArH), 7.50(t, 2H, J=7.72Hz, ArH), 7.45(s, 1H, =CH). ¹³C-NMR(400MHz, CDCl₃), δ(ppm): 215.889, 173.236, 136.260, 132.488, 131.937, 129.929, 127.210. HR-MS: Calcd. For C₉H₆S₃[M+H]⁺: 210.9704, Found: 210.9704.

### Example 10: Synthesis of 5-(2-naphthyl)-3H-1,2-dithiacyclopentene-3-thione (YC1-10)

Based on the synthesis method of YC1-1, YC1-10 was obtained using methyl 2-naphthoyl acetate, phosphorus pentasulfide and sulfur, with a yield of 42%. m.p.137.1∼138.3°C. ¹HNMR(400Hz, CDCl₃), δ(ppm): 8.20(s, 1H, Ar), 7.88 - 7.93(m, 2H, ArH), 7.90(d, 1H, J=7.32Hz, ArH), 7.68 (dd, 1H, J=1.77, 8.56Hz, ArH), 7.62 (dt, 2H, J=7.01Hz, ArH), 7.58(s, 1H, =CH). ¹³C-NMR (400MHz, CDCl₃), δ(ppm): 215.752, 173.255, 136.385, 135.072, 133.258, 129.940, 129.251, 129.196, 128.756, 128.280, 127.924, 127.558, 123.711. HR-MS: Calcd_{.} For C₁₃H₈S₃[M+H]⁺: 260.9861, Found: 260.9862.

### Example 11: Synthesis of 4-(3H-1,2-dithio-3-thioketone 5-yl)phenyl 4-isobutyl-α-methyl-phenylacetate (YC2-1).

Ibuprofen (0.206 g, 1.0 mmol), dicyclohexylcarbodiimide (0.206 g, 1.0 mmol), ADT-OH (0.226 g, 1.0 mmol) and a catalytical amount of 4-dimethylamino pyridine were added to 10 ml dichloromethane, and stirred at room temperature for 2 h. After filtration, it was washed by water for 3 times, and the organic phase was dried over anhydrous MgSO₄. After the solvent was evaporated to dryness, column chromatography was carried out using petroleum ether : ethyl acetate = 20 : 1 as the eluent, to obtain a reddish-brown solid with a yield of 94.6% and m.p.63.0-64.0°C. ¹H NMR (400MHz, CDCl₃), δ(ppm): 7.63(d, 2H, *J =* 8.6 Hz, ArH), 7.38(s, 1H, =CH), 7.29(d, 2H, *J* = 7.9 Hz, ArH), 7.15(m, 4H, ArH), 3.96(q, 1H, CH), 2.48(d, 2H, CH₂), 1.87(td, 1H, CH), 1.62(d, 3H, CH₃), 0.91(d, 6H, CH₃).IR(KBr, cm⁻¹): 1740.4 (C=O), 1631.8, 1597.1, 1521.8, 1492.9 (C=C). HR-MS: Calcd. For C₂₂H₂₂O₂S₃ 414.0782, Found: 414.0799.

### Example 12: Synthesis of 4-(3H-1,2-dithio-3-thioketone-5-yl)phenyl 2-acetoxy benzoate (YC2-2)

Based on the synthesis method of YC2-1, YC2-2 was prepared using aspirin and ADT-OH to obtain a red solid, with a yield of 80.0% and m.p. 125.0-126.0°C. ¹H NMR (400MHz, CDCl₃), δ(ppm): 8.23(m, 1H, ArH), 7.73(d, 2H, *J* = 8.5 Hz, ArH), 7.68(m, 1H, ArH), 7.42(m, 2H, ArH & =CH), 7.33(d, 2H, *J=* 8.6 Hz, ArH), 7.20(d, 1H, *J=* 8.0 Hz, ArH), 2.32(s, 3H, CH₃). IR(KBr, cm⁻¹): 1752.6 (C=O), 1746.8 (C=O), 1645.3, 1596.5, 1573.1, 1489.6 (C=C), 1196.1 (C=S). HR-MS: Calcd. For C₁₈H₁₂O₄S₃ 387.9898, Found: 387.9899.

### Example 13: Synthesis of 4-(3H-1,2-dithio-3-thioketone-5-yl)phenyl pyridine-3-carboxylate (YC2-3)

Based on the synthesis method of YC2-1, YC2-3 was prepared using nicotinic acid and ADT-OH to obtain a red solid, with a yield of 57% and m.p. 165.5-177.3°C. ¹H-NMR(400MHz, CDCl₃), δ(ppm): 9.28(d, 1H, J=1.1Hz, ArH), 8.91-8.92(m,1H, ArH), 8.49(d, 1H, J=7.9Hz, ArH), 8.04(d, 2H, J=8.6Hz, ArH), 7.86(s, 1H, ArH), 7.66-7.69(m, 1H, ArH), 7.54(d, 2H, J=8.7Hz, ArH).

### Example 14: Synthesis of 4-(3H-1,2-dithio-3-thioketone-5-yl)phenyl 3-benzoyl-phenylacetate (YC2-4)

Based on the synthesis method of YC2-1, YC2-4 was prepared using ketoprofen and ADT-OH to obtain a red solid, with a yield of 92.5% and m.p. 107.7-109.2°C. ¹H NMR(400MHz,CDCl₃), δ(ppm): 7.87(s, 1H, ArH), 7.82(d, 2H, *J=* 8.3 Hz, ArH), 7.74(d, 1H, *J=* 7.6 Hz, ArH), 7.66(s, 1H, ArH), 7.64(m, 2H, ArH), 7.61(d, 1H, *J=* 7.3 Hz, ArH), 7.50(m, 3H, ArH), 7.39(s, 1H, =CH), 7.17(d, 2H, ArH), 4.08(q, 1H, CH),1.68(d, 3H, CH₃). IR(KBr, cm⁻¹): 1757.2 (C=O), 1654.9, 1626.0, 1597.0, 1489.0 (C=C), 1168.9 (C=S). HR-MS: Calcd. For C₂₅H₁₉O₃S₃ 4862.0418, Found: 462.0411.

### Example 15: Synthesis of 4-(3H-1,2-dithio-3-thioketone-5-yl)-phenyl 2-[(2,6-dichlorophenyl)amino]phenylacetate(YC2-5)

Based on the synthesis method of YC2-1, YC2-5 was prepared using diclofenac and ADT-OH to obtain a red solid, with a yield of 85.6% and m.p. 151.0-153.0°C. ¹H NMR (400MHz, CDCl₃), δ(ppm): 7.68 (d, 2H, *J* = 8.7 Hz, ArH), 7.40 (s, 1H, =CH), 7.35 (d, 3H, *J* = 8.0 Hz, ArH), 7.25 (s, 1H, ArH), 7.20 (t, 2H, *J* = 7.2 Hz, ArH), 7.02 (m, 2H, ArH), 6.65 (s, 1H, NH), 6.61 (d, 1H, *J* = 8.0 Hz, ArH), 4.09 (s, 2H, CH₂).

### Example 16: Effect of 3H-1,2-dithiocyclopentene-3-thione compounds on HT22 hippocampal neuron cell line

### 1. Materials and equipments

Mouse Hippocampal neuron cell line HT22, 24-well culture plate (Corning), glutamic acid (Glutamate, Sigma), DMEM culture medium (GIBCO), fetal bovine serum (GIBCO), 3-(4,5-dimethylthiazol-2-yl)-2,5-diphenyltetrazolium bromide (MTT, Sigma), dimethyl sulfoxide (Sinopharm Group. Co. Ltd.), and microplate reader (TECAN).

### 2. Experimental methods

2.1. Cell culture: HT22 cells were taken out of the liquid nitrogen container, and thawed quickly in a water-bath at 37°C. After that, the outside wall of cryopreservation tube was disinfected by alcohol wipes, and it was then placed in a clean bench. The cells were transferred to a centrifuge tube containing 10 ml medium and centrifuged at 1000 rpm for 3 min. After centrifugation, the supernatant was discarded, and 10 mL DMEM containing 10% FBS was added to the centrifuge tube. The mixture was vigorously pipetted to prepare a cell suspension. The cell suspension was inoculated to a 100×20 mm Petri dish. Cells were cultured at 37°C and 5% CO₂. When the cell density reached confluent, the cells were digested and seeded onto the wells of a 24-well plate at the density of 2×10⁴/well.
2.2. Treatment and groups: after the cells were cultured in the 24-well plate for 24 hours, 5 mM glutamate was added together with the compound each synthesized in Example 1-10 or Example 13 at a final concentration of 1, 10, 50, or 100 µM. DMSO served as vehicle and was added to the cells of the control group at an equal volume. The viability of the cells was detected by MTT method 24 h after the treatment.
2.3. Detection by MTT: 50µL 0.5 mg/ml MTT was added to each well 24 h after the treatment of cells. The cells were further incubated at 37°C and 5% CO₂ for 4 h. Subsequently, the supernatant was discarded. The cells were lysed by adding 500µL dimethyl sulfoxide (DMSO). The absorbance was detected at 570 nm by a microplate reader, which is positively correlated with survival rate of cells. The survival rate of the cells in the treatment groups was expressed as the percent of the survival rate of the cells without glutamate treatment. All data were expressed as mean ± standard deviation (SD). Statistical analysis was carried out for all data using SPSS 17.0. The difference between the groups was tested by one way anova. P<0.01 was considered as very significant difference. The statistical results were listed in table 1.

**Table. 1 Effect of 3H-1,2-dithiocyclopentene-3-thione compounds on the survival of HT22 cells in the glutamate-induced neural oxidative stress injury model**

| Compound | Concentration range for protection (µM) | Survival rate % |
|---|---|---|
| Glutamate | / | 24.1±1.4 |
| YC1-1 | 10-100 | 52.2±2.97-103.89±3.66 |
| YC1-2 | 10-100 | 75.88±0.17-93.76±0.86 |
| YC1-3 | 10-100 | 85.92±0.01-114.74±0.36 |
| YC1-4 | 10-100 | 56.53±0.99-68.69±0.89 |
| YC1-5 | 1-100 | 72.87±0.18-109.46±0.60 |
| YC1-6 | 1-100 | 50.32±0.02-106.33±0.81 |
| YC1-7 | 1-100 | 55.44±0.60-116.38±0.07 |
| YC1-8 | 10-100 | 71.37±1.86-99.24±1.27 |
| YC1-9 | 1-100 | 76.39±0.42-133.81±0.55 |
| YC1-10 | 1-100 | 46.39±0.07-94.17±0.39 |
| YC2-3 | 50-100 | 42.01±0.09-82.98±0.98 |

As shown in Table 1, 5 mM glutamate significantly led to the death of cells, and the survival rate of the HT22 cells treated with glutamate was only 24% of the control group without glutamate treatment. Glutamate-induced HT22 cell death was significantly inhibited by the compounds synthesized in Example 1-11 at a final concentration in the range from 1 to 100µM, and the survival rate was thus significantly enhanced, which was higher than 42% for each compound, indicating a direct protection of 3H-1,2-dithiocyclopentene-3-thione compounds on neurons against glutamate-induced neural oxidative stress injury model.

### Example 17: Effect of 3H-1,2-dithiocyclopentene-3-thione compounds on the lipopolysaccharide (LPS) induced inflammatory response of microglia (brain inflammatory cell)

### 1. Materials and equipments

Brain microglia cell line BV-2, 96-well culture plate (Corning, USA), DMEM medium (Hyclone, USA), fetal bovine serum (FBS) (GIBCO, USA), dimethyl sulfoxide (DMSO) (SIGMA, USA), Griess reagent (SIGMA, USA), sodium nitrite (ChemNet, China), microplate reader (BIO-RAD, USA)

### 2. Experimental methods:

2.1. Cell cultures: After taken out of the liquid nitrogen container, BV-2 cells were quickly placed in warm water at 37°C and gently shaken for thawing. Subsequently, it was centrifuged at 1000 rpm for 3 min, and the supernatant was discarded. The cells were then cultured in DMEM containing 10% FBS, and passage was carried out when 60%-70% of confluence was achieved in the Petri dish. When 60%-70% of confluence of the passaged cells was achieved, the cells were harvested by centrifugation at 1000 rpm for 3 min. The cells were then stained by trypan blue and counted under a microscope by globulimeter. After cell counting, it was diluted into a suspension containing 150 to 200 thousand of cells/ml, which was then plated in the 96-well plate (15 to 20 thousand of cells/ml).
2.2. Treatment and groups: after the cells were cultured in the 24-well plate for 24 h, 200 ng/mL LPS was added together with compound YC1-1 of Example 1, YC1-8 of Example 8 and YC2-2 of Example 12 at a final concentration of 1, 10, 50, or 100µM. DMSO served as vehicle and was added to the cells of control group at the equal volume. The cells were further incubated in an incubator.
2.3. The amount of nitrite produced was detected 24 h after the atreatment, to characterize the inflammatory response of BV2 cells induced by LPS and the inhibitory effect of compound YC1-1 of Example 1 and compound YC1-8 of Example 8 on the LPS-induced inflammatory response.

Detection method of nitrite: 5µL supernatant was taken out of the wells of 96-well plate, and added to the corresponding wells of another clean 96-well plate. Subsequently, 50µL Griess reagent was added to each well, and the OD value was detected at 570 nm. The amount of nitrite was calculated based on the standard curve. The result was expressed as the relative yield of nitrite, wherein the relative yield of the control group treated only by LPS was designated as 1. All data were expressed as mean ± standard deviation (SD). Statistical analysis was carried out for all data using SPSS 17.0. The difference between each group was tested by one way anova. P<0.01 was considered as very significant difference. The statistical results were listed in Fig. 1 and Fig. 2.

The result regarding nitrite production showed that, robust inflammatory response of BV2 cells was induced by LPS, while the LPS induced inflammatory response of BV2 cells can be significantly inhibited by compound YC1-1 of Example 1 (Fig. 1), compound YC1-8 of Example 8 (Fig. 2) and compound YC2-2 of Example 12 (Fig. 3) at a concentration in a range from 10-100µM.

### Example 18: Protective effect of 3H-1,2-dithiocyclopentene-3-thione compounds against cerebral ischemia in mouse middle cerebral artery occlusion model

### 1. Materials and equipments

Male ICR mice (body weight of 30±2.0 g) were purchased from Shanghai Laboratory Animal Center, Chinese Academy of Sciences. The mice were raised at room temperature and 12 h cycle of light and dark, with free access to food and water.

2,3,5-triphenyl tetrazolium chloride (TTC) (Sigma), 0.9% physiological saline (Anhui Shuanghe Pharmaceutical Co., Ltd), isoflurane (Shandong Keyuan Pharmaceutical Co., Ltd.), 4% paraformaldehyde (Sinopharm Chemical Reagent Beijing Co., Ltd), XTS-4A operating microscope (Zhenjiang Zhongtian optical instrument Co., Ltd), dimethyl sulfoxide (DMSO), corn oil (Sigma), thermostatic water bath SSW-600-2S (Shanghai Boxun Industry & Commerce Co., Ltd), digital camera (Canon), laser-Doppler blood flowmeter PeriFlux system 5000 (PERIMAD AB).

### 2. Experimental methods

2.1. Middle cerebral artery occlusion (MCAO) model: the middle cerebral artery occlusion (MCAO) model was performed by reference to Longa's method or other modified suture-occluded method. The specific experimental procedures were as follows: after anaesthetized by isoflurane, the mouse was fixed in supine position, and the skin of neck and head was sterilized using iodine. The probe of laser-Doppler blood flowmeter was fixed at the midpoint of the line between the ear and the eye on the right side of the mouse to monitor the real-time perfusion in the cerebral cortex. A 1.5 cm incision was made in the middle of the neck to expose common carotid artery on the right side. Tissues were separated under an operating microscope layer by layer. The common carotid artery was first separated which was temporarily ligated 0.5 cm away from the distal end using 5-0 suture.. Then, the external carotid artery and internal carotid artery on the right side were subsequently separated and the external carotid artery was ligated 0.5 cm away from the distal end using 5-0 suture. At the same time, the internal carotid artery was temporarily clamped using a mini vascular clamp.. Subsequently, a small incision was made by an ophthalmic scissor on the inner side of the ligation at the distal end of the external carotid artery. To occlude the common carotid artery, one of the ends of the suture was slightly enlarged by heating, coated by silica gel, and sterilized using alcohol. The length of the suture was about 2 cm, and the diameter of the enlarged end was 0.22±0.01 cm. The suture was held using a fine forceps and inserted into the incision on the external carotid artery..). Once the suture passed through the incision of the external carotid artery, the ligation was lifted at the distal end of external carotid artery until the enlarged end of the suture entered the bifurcation of the common carotid artery. The suture was then tightened,and the vascular clamp on the internal carotid artery was opened. The suture was slowly advanced from the external carotid artery into the internal carotid artery through the bifurcation until slight resistance was felt, at which time a sudden sharp decrease in cortical perfusion was observed on laser-Doppler blood flowmeter (the perfusion was decreased to less than 25% of the baseline), indicating that the enlarged end of the suture arrived at the beginning of the middle cerebral artery and occluded the blood perfusion. The starting time for occlusion was recorded. For reperfusion, the suture was withdrawn 60 min after middle cerebral artery occlusion. The rectal temperature was maintained at 37.0±0.5°C by a heating blanket during and after the surgery, until the animal was revived.
2.2 Animal groups and drug administration: the animals were randomly divided into 3 groups, including control group treated with vehicle, YC1-8 group (100 mg/kg) and YC1-8 group (50 mg/kg). There were 8 mice in each group. 12 mg YC1-8 was initially dissolved by 80 µL DMSO, and then diluted by 920µL corn oil to prepare a solution with the final concentration of 12 mg/mL. One half of dosage of compound YC1-8 was initially administrated at 3 h of reperfusion after ischemia by intraperitoneal injection, while the other half of dosage was administrated at 10 h of reperfusion after ischemia. For the control group, the same volume of DMSO-containing corn oil was administrated to the mice at 3 h and 10 h.
2.3. Detection of the cerebral infarct volumes: 24 h after reperfusion following mouse middle cerebral artery occlusion (MCAO), the mice were sacrificed under anesthetized and the brain was collected. After the brain tissue was stripped off, the olfactory bulb and cerebellum were removed. A series of 5 coronary sections of brain were made at an interval of 1 mm, which were then placed in 2% TTC solution at 37°C under darkness in a warm bath. The sections were turned over every 15 min, with a total duration of 30 min. The size of cerebral infarction was determined by TTC staining method. Due to the reduction of TTC by catalase in mitochondrion,, normal brain tissue was red, and the ischemic infarct area was white ,. After staining, the sections were fixed in 4% paraformaldehyde solution for 24h, and then photographed by a digital camera. The results were expressed as the percent of the infarct volumes on the ischemic side to the corresponding structures on the contralateral side. The percent of infarction volume was calculated and analyzed using software. All data were expressed as mean ± standard deviation (SD). The difference between each group was compared by t test. P<0.05 was considered as significant difference. The statistical results were listed in Fig. 4.

As shown in Fig. 4, at 24 h after middle cerebral artery occlusion, the infarction volumes in cerebral cortex, striatum and hemisphere were significantly reduced by injection of 100 mg/kg and 50 mg/kg YC1-8, as compared to the control mice treated with vehicle. The results suggested that there was a remarkable protective effect of YC1-8 on the ischemic brain injury resulted from middle cerebral artery occlusion.

The above Examples are only described for understanding the methods and principal concepts of the present invention. It should be noted that several improvements and modifications can be made to the present invention by the person of ordinary skill in the art without departing from the principles of the present invention. These improvements and modifications should also be regarded as in the scope of the claims of the present invention.

## Claims

1. 3H-1,2-dithiocyclopentene-3-thione compounds, which are **characterized in that** the compounds have the structure of formula I or formula II, 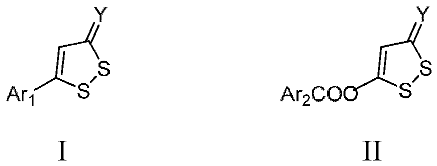 wherein Y is selected from S, O or NHOH; Ar₁ is selected from naphthyl, phenyl, thiophene, furan, pyrrole, imidazole, thiazole, pyrazole, and pyridine; the substituent on Ar₁ is a mono-substituent, di-substituent or tri-substituent, and the substituents are independently from each other selected from -H, -OH, -NH₂, -X, -SO₂CH₃, -SO₂NH₂, -CN, -NO₂, -COOH, Ar- or R-, RO-, XR-, RNH-, NH₂R-, NO₂OR-, HOOCR-, wherein X is F, Cl, Br, or I, and R represents an alkyl of 1-8 carbon atoms; Ar₂COO is selected the carboxylic residue from nicotinic acid, aspirin, diclofenac, ketoprofen or ibuprofen.

2. The compounds according to claim 1, which are **characterized in that**, the compounds are selected from 5-*p*-hydroxyphenyl-3H-1,2-dithiacyclopentene-3-thione, 5-*p*-fluorophenyl-3H-1,2-dithiacyclopentene-3-thione, 5-(2-thienyl)-3H-1,2-dithiacyclopentene-3-thione, 5-*p*- methylphenyl -3H-1,2-dithiacyclopentene-3-thione, 5-(2,4-dichloro-5-fluorophenyl)-3H-1,2-dithiacyclopentene-3-thione, 5-(3,4-methylenedioxyphenyl)-3H-1,2-dithiacyclopentene-3-thione, 5-(2,4-dimethoxyphenyl)-3H-1,2-dithiacyclopentene-3-thione, 5-*p*-methoxyphenyl-3H-1,2-dithiacyclopentene-3-thione, 5-phenyl-3H-1,2-dithiacyclopentene-3-thione, 5-(2-naphthyl)-3H-1,2-dithiacyclopentene-3-thione, 4-(3H-1,2-dithio-3-thioketone 5-yl)phenyl 4-isobutyl-α-methyl-phenylacetate, 4-(3H-1,2-dithio-3-thioketone-5-yl)phenyl 2-acetoxy benzoate, 4-(3H-1,2-dithio-3-thioketone-5-yl)phenyl pyridine-3-carboxylate, 4-(3H-1,2-dithio-3-thioketone-5-yl)phenyl 3-benzoyl-phenylacetate or 4-(3H-1,2-dithio-3-thioketone-5-yl)-phenyl 2-[(2,6-dichlorophenyl)amino]phenylacetate.

3. Use of 3H-1,2-dithiocyclopentene-3-thione compounds and the pharmaceutically acceptable salts thereof in preparation of medicines for the prevention or treatment of cerebral apoplexy diseases.

4. The use according to claim 3, which is **characterized in that** the compounds have the structure of formula I or formula II, 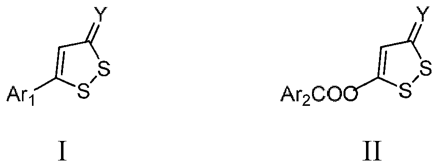 wherein Y is selected from S, O or NHOH; Ar₁ is selected from naphthyl, phenyl, thiophene, furan, pyrrole, imidazole, thiazole, pyrazole, and pyridine; the substituent on Ar₁ is a mono-substituent, di-substituent or tri-substituent, and the substituents are independently from each other selected from -H, -OH, -NH₂, -X, -SO₂CH₃, -SO₂NH₂, -CN, -NO₂, -COOH, Ar- or R-, RO-, XR-, RNH-, NH₂R-, NO₂OR-, HOOCR-, wherein X is F, Cl, Br, or I, and R represents an alkyl of 1-8 carbon atoms; Ar₂COO is selected from the carboxylic residue from nicotinic acid, diclofenac, ketoprofen or ibuprofen.

5. The use according to claim 3, which is **characterized in that** the 3H-1,2-dithiocyclopentene-3-thione compounds are selected from 5-*p*-hydroxyphenyl-3H-1,2-dithiacyclopentene-3-thione, 5-*p*-fluorophenyl-3H-1,2-dithiacyclopentene-3-thione, 5-(2-thienyl)-3H-1,2-dithiacyclopentene-3-thione, 5-*p*- methylphenyl -3H-1,2-dithiacyclopentene-3-thione, 5-(2,4-dichloro-5-fluorophenyl)-3H-1,2-dithiacyclopentene-3-thione, 5-(3,4-methylenedioxyphenyl)-3H-1,2-dithiacyclopentene-3-thione, 5-(2,4-dimethoxyphenyl)-3H-1,2-dithiacyclopentene-3-thione, 5-*p*-methoxyphenyl-3H-1,2-dithiacyclopentene-3-thione, 5-phenyl-3H-1,2-dithiacyclopentene-3-thione, 5-(2-naphthyl)-3H-1,2-dithiacyclopentene-3-thione, 4-(3H-1,2-dithio-3-thioketone 5-yl)phenyl 4-isobutyl-α-methyl-phenylacetate, 4-(3H-1,2-dithio-3-thioketone-5-yl)phenyl 2-acetoxy benzoate, 4-(3H-1,2-dithio-3-thioketone-5-yl)phenyl pyridine-3-carboxylate, 4-(3H-1,2-dithio-3-thioketone-5-yl)phenyl 3-benzoyl-phenylacetate or 4-(3H-1,2-dithio-3-thioketone-5-yl)-phenyl 2-[(2,6-dichlorophenyl)amino]phenylacetate.

6. The use according to claim 3, which is **characterized in that**, the cerebral apoplexy is selected from the cerebral apoplexy resulted from cerebral ischemia, cerebral embolism or cerebral hemorrhage.

7. The use according to claim 3, which is **characterized in that** the cerebral apoplexy is selected from one or more of cerebral ischemia, cerebral thrombosis, cerebral embolism, cerebral infarction, lacunar infarction, transient ischemic attack, cerebral hemorrhage, cerebral arteriosclerosis, or cerebral ischemia due to sudden cardiac arrest.

8. A pharmaceutical formulation for the prevention or treatment of cerebral apoplexy diseases, which is **characterized in that** the formulation comprises an effective amount of 3H-1,2-dithiocyclopentene-3-thione compounds or the pharmaceutically acceptable salts thereof and the pharmaceutically acceptable adjuvants.

9. The pharmaceutical formulation according to claim 8, which is **characterized in that** the pharmaceutical formulation is selected from but not limited to capsule, microcapsule, tablet, granule, dispersible powder, injection, liposome, oral solution, intravenous injection, intramuscular injection or the formulation that can be directly applied to the site of cerebral ischemia.
